## Europäisches Patentamt
## European Patent Office
## Office européen des brevets

(19)

(11) Numéro de publication: **0 133 403**
A2

(12)

# DEMANDE DE BREVET EUROPEEN

(21) Numéro de dépôt: 84401593.3

(22) Date de dépôt: 30.07.84

(51) Int. Cl.⁴: **C 12 N 15/00**, C 12 P 21/02,
C 07 K 7/04, C 07 H 21/04,
C 12 N 1/20, A 61 K 39/05,
A 61 K 39/385
// (C12N1/20, C12R1/16, 1/19)

(30) Priorité: **29.07.83 FR 8312597**

(43) Date de publication de la demande: **20.02.85**
**Bulletin 85/8**

(84) Etats contractants désignés: **AT BE CH DE GB IT LI LU NL SE**

(71) Demandeur: **GROUPEMENT DE GENIE GENETIQUE (G3), 28, rue du Docteur Roux, F-75015 Paris (FR)**
Demandeur: **INSTITUT PASTEUR, 25-28, rue du Docteur Roux, F-75724 Paris Cedex 15 (FR)**
Demandeur: **INSTITUT NATIONAL DE LA SANTE ET DE LA RECHERCHE MEDICALE (INSERM), 101, rue de Tolbiac, F-75654 Paris Cedex 13 (FR)**

(72) Inventeur: **Kaczorek, Michel, 6, rue Mayran, F-75009 Paris (FR)**
Inventeur: **Tiollais, Pierre, 16, rue de la Glacière, F-75013 Paris (FR)**
Inventeur: **Chenciner, Nicole, 11, Quai Bourbon, F-75004 Paris (FR)**
Inventeur: **Streeck, Rolf E., 17 bis, avenue Foch, F-75116 Paris (FR)**
Inventeur: **Boquet, Patrice, 1, rue du Puits Georget, F-94000 Creteil (FR)**

(74) Mandataire: **Gutmann, Ernest et al, Cabinet Plasseraud 84, rue d'Amsterdam, F-75009 Paris (FR)**

(54) Séquence nucléotidique codant pour le peptide signal de la toxine diphtérique, vecteur contenant cette séquence nucléotidique, leur application à la transformation de micro-organismes et compositions peptidiques obtenues.

(57) L'invention concerne un fragment d'ADN contenant au moins 75 paires de nucléotides et en contenant au plus 1000, notamment au plus 375, caractérisé en ce qu'il contient une séquence basale de nucléotides codant pour la séquence peptidique suivante: 
MET SER ARG LYS LEU PHE ALA SER ILE LEU ILE GLY
ALA LEU LEU GLY ILE GLY ALA PRO PRO SER ALA HIS ALA
MET SER ARG LYS LEU PHE ALA SER ILE LEU ILE GLY ALA LEU LEU GLY ILE GLY ALA PRO PRO SER ALA HIS ALA

EP 0 133 403 A2

ACTORUM AG

Séquence nucléotidique codant pour le peptide signal de la toxine diphtérique, vecteur contenant cette séquence nucléotidique, leur application à la transformation de micro-organismes et compositions peptidiques obtenues.

L'invention concerne une séquence nucléotidique, de préférence un ADN, codant pour le peptide signal de la toxine diphtérique, les vecteurs contenant cette séquence nucléotidique, leur application à la transformation de micro-organismes et les compositions peptidiques susceptibles d'être obtenues.

On sait que le gène de la toxine diphtérique est localisé dans le génome de certains Corynebactériophages, comme $\beta^{tox+}$, $\omega^{tox+}$. Certaines séquences protéiques de la toxine ont déjà été déterminées, du moins dans la souche PW8 de Corynebacterium diphteriae.

On connaît des mutants du phage β codant pour une toxine présentant globalement la même structure moléculaire apparente, dont le fragment A a cependant perdu son activité enzymatique et dont le fragment B ne possède plus qu'une capacité réduite de reconnaissance des récepteurs membranaires des cellules que la toxine active est apte à envahir, ou vice versa. Cette protéine ne peut cependant être distinguée, sur le plan sérologique, de la toxine active. On mentionnera parmi les Corynebactériophages connus contenant des gènes de ce type, le Corynebactériophage $\beta_c 228$ obtenu par mutagénèse d'un phage sauvage $\beta^{tox+}$ par traitement avec la N-méthyl-N'-nitro-N-nitrosoguanidine (NG) d'une culture de Corynebacterium $C_7 \beta^{tox+}$ (UCHIDA, T., PAPPENHEIMER, Jr. A. M., et GREANY, R., J. Biol. Chem. 248, 3838-3844 (1973)).

Il a également été mentionné dans la littérature que la production cellulaire de la toxine diphtérique impliquait la synthèse d'un précurseur contenant, outre la toxine elle-même, un peptide signal dénommé "peptide S". Ce peptide semble jouer un rôle essentiel au niveau de la capacité de la toxine synthétisée dans Corynebacterium diphteriae de traverser les membranes de ces cellules et d'être excrétée dans le milieu extérieur, après clivage enzymatique du peptide S par la bactérie.

L'hypothèse de l'existence initiale de ce peptide S dans le précurseur de la toxine diphtérique avait déjà été envisagée par SMITH et Coll. (J. Bact. 141, 184-189 (1980)). Cette hypothèse s'appuyait sur des observations faites au cours d'essais de synthèse in vitro de la toxine diphtérique à partir de l'ARN messager des polysomes membranaires de Corynebacterium diphteriae dans un extrait de E. coli complété avec les acides aminés naturels de base et des ingrédients nécessaires à de telles synthèses.

Le fait de disposer de l'ARN messager permet également de localiser le gène de la toxine diphtérique dans le génome du phage β dans des essais d'hybridation avec ce dernier. Mais l'étude et la confirmation de l'existence effective de ce peptide signal et, par conséquent, de la "séquence S" d'ADN codant pour le peptide S, n'avaient pas été réalisées jusqu'à ce jour.

L'invention découle de la découverte qu'il était possible d'induire dans E. coli la production d'une protéine susceptible d'être reconnue par des anticorps préalablement formés contre la toxine diphtérique ou une anatoxine diphtérique, après transformation préalable de cet E. coli par un plasmide dans lequel avait été insérée une séquence excisée à partir du génome du phage β. Cette séquence était suffisamment étendue pour que l'on puisse présumer qu'elle contenait aussi la séquence d'ADN codant pour le peptide signal, ainsi que la séquence en aval de la précédente contenant les éléments de régulation de la transcription et de la traduction du gène de la toxine diphtérique. C'est ainsi qu'une culture de E. coli préalablement transformée par des plasmides (pTD44 et pTD76), résultant de la modification de pBR 322 par des insérats contenant le gène codant pour la toxine diphtérique CRM 228, devenait capable de synthétiser un polypeptide reconnu immunologiquement par des anticorps formés contre cette toxine. Dans ces plasmides, mentionnés à titre d'exemples, l'insérat (dans le site BamHI de pBR 322) était constitué par un fragment délimité par des extrémités BamHI, de 3,9 kilobases (kb). Ce fragment avait lui-même été obtenu à partir du génome du phage $\beta_c 228$, par hydrolyse de ce dernier par l'enzyme de restriction BamHI, isolement des fragments de restriction obtenus et hybridation de ceux-ci avec une sonde d'ADN radioactive complémentaire d'un ARN messager de la toxine, lui-même préalablement isolé des polysomes membranaires d'une souche de Corynebacterium diphteriae secrétrice de toxine. Cette synthèse de CRM 228 a été possible, indépendamment de l'orientation de l'insérat dans le plasmide modifié utilisé pour la transformation des souches productrices. En particulier, les plasmides pTD 44 et pTD 76 ne se distinguaient entre eux que par les orientations dans des sens respectivement opposés des susdits insérats BamHI.

L'expression possible dans E. coli de la toxine CRM 228, quelle que soit l'orientation de l'insérat dans les plasmides utilisés pour la transformation, témoignait donc de ce que cette expression s'était effectuée sous le contrôle des éléments de régulation normaux de la transcription et de la traduction de la toxine diphtérique dans Corynebacterium diphteriae, donc de son promoteur naturel, lequel était vraisemblablement inclus dans le susdit fragment BamHI de 3,9 kb. Cette présomption a été confirmée par les essais décrits plus loin dans les exemples.

En d'autres termes, la machinerie cellulaire de E. coli s'est révélée capable de reconnaître les séquences de la transcription et de la traduction de la toxine diphtérique originaire du phage β. Ce résultat est d'autant plus remarquable que les Corynae bacteriae et les souches de E. coli sont phylogénétiquement éloignées les unes des autres. Les Corynae bacteriae sont en effet des cellules Gram$^+$ à membrane unique alors que les E. coli sont des cellules Gram$^-$ à double membrane.

Cette découverte est à l'origine de l'invention, en ce qu'elle a permis de compléter l'analyse séquentielle du gène codant pour la toxine diphtérique. En outre, elle a conduit aux divers éléments de l'invention, à savoir un peptide, hybride ou non, contenant notamment une séquence peptidique identique ou semblable à la séquence peptidique signal (peptide S), à des fragments d'ADN et à des ADN hybrides contenant une séquence nucléotidique codant pour le susdit peptide et à des vecteurs la mettant en jeu et permettant la transformation aisée de micro-organismes de divers types.

L'invention concerne plus particulièrement un fragment d'ADN contenant au moins 75 paires de nucléotides, et en contenant au plus 1000, de préférence au plus 375, caractérisé en ce qu'il contient une séquence basale de nucléotides codant pour la séquence peptidique suivante :

MET SER ARG LYS LEU PHE ALA SER ILE LEU ILE GLY ALA LEU LEU GLY ILE

GLY ALA PRO PRO SER ALA HIS ALA

Elle concerne plus particulièrement un fragment contenant la séquence basale suivante de nucléotides :

GTG AGC AGA AAA CTG TTT GCG TCA ATC TTA ATA GGG GCG CTA CTG GGG ATA

GGG GCC CCA CCT TCA GCC CAT GCA

L'invention concerne encore un fragment d'ADN préféré répondant à l'une ou l'autre des définitions sus-indiquées, ce fragment contenant une séquence mixte comportant une seconde séquence contenant un promoteur, en sus de la susdite séquence basale et en amont de celle-ci, vis-à-vis de la direction de lecture, la séquence basale étant sous le contrôle de ce promoteur.

De  p r é f é r e n c e ,                    cette séquence mixte     présente la structure nucléotidique suivante :

ATCTTTGCGG TGTGGTACAC CTGATCTGGT CCGGTTCATG TTGTGGTGGT CAACGCTGGG
-300                                                      -250

GTAACCGGCG TTGCGTATCC AGTGGCTACA CTCAGGTTGT AATGATTGGG ATGATGTACC
                                                   -200

TGATCTGAGA GCGATTAAAA ACTCATTGAG CAGTAGGTCC CGATTGGTTT TTGCTAGTGA
                                        -150

AGCTTAGCTA GCTTTCCCCA TGTAACCAAT CTATCAAAAA AGGGCATTGA TTTCAGAGCA
                              -100

CCCTTATAAT TAGGATAGCT TTACCTAATT ATTTTATGAG TCCTGGTAAG GGGATACGTT
                -50                                                    -1

GTG AGC AGA AAA CTG TTT GCG TCA ATC TTA ATA GGG GCG CTA CTG GGG ATA

GGG GCC CCA CCT TCA GCC CAT GCA

L'invention concerne également des ADN hybrides contenant un fragment, tel que l'un de ceux définis ci-dessus, associés à une séquence hétérologue vis-à-vis de l'ADN du gène du phage β. Par séquence d'ADN hétérologue, il faut ici entendre une séquence non hybridable avec le génome du phage β, et plus particulièrement une séquence d'ADN appartenant au patrimoine génétique d'un micro-organisme distinct du phage β.

Plus particulièrement encore cette séquence d'ADN hétérologue correspond à l'ADN d'un vecteur apte à transformer un micro-organisme déterminé.

Un ADN hybride préféré de l'invention est caractérisé en ce qu'il consiste en un vecteur apte à transformer un micro-organisme déterminé et comportant un promoteur susceptible d'être reconnu par ledit micro-organisme déterminé, ce vecteur étant modifié par l'insertion dans un site en amont de ce promoteur et sous le contrôle de celui-ci, de la séquence basale. Ce promoteur peut être celui de la susdite séquence mixte, définie ci-dessus. Il peut encore s'agir d'un promoteur distinct, non originaire d'un gène de toxine diphtérique.

Avantageusement, ce vecteur est de préférence du type plasmide et est choisi parmi ceux qui sont aptes à transformer une souche de E. coli ou une souche de Corynebacterium.

Le susdit promoteur peut être choisi parmi ceux qui sont reconnus par la machinerie cellulaire du micro-organisme qui est à transformer, par exemple le promoteur de l'opéron lactose, dans le cas où le micro-organisme à transformer est constitué par un E. coli. Il est encore avantageusement constitué par le promoteur sous le contrôle duquel la susdite séquence basale est transcrite et exprimée dans Corynebacterium. Ce cas est notamment celui où l'ADN hybride ou le vecteur considéré contient la séquence mixte telle que ci-dessus définie.

Un tel vecteur présente un intérêt important, surtout lorsqu'il est à son tour modifié par un insérat supplémentaire également placé sous le contrôle de ce promoteur et susceptible d'être transcrit et/ou traduit en une protéine déterminée, dont la production est recherchée, notamment dans un micro-organisme transformable par ce vecteur.

Les susdites séquences basales ou séquences mixtes sont donc avantageusement introduites dans tous types de vecteurs, et notamment plasmides connus pour leur capacité à transformer E. coli. Une catégorie de vecteurs particulièrement intéressante, modifiés par les insérats sus-définis sont ceux qui sont aptes à transformer les Corynebacteriae eux-mêmes. A titre d'exemple de tels vecteurs, on mentionnera des plasmides ou ADN phagiques dont la capacité à transformer des micro-organismes appartenant à l'espèce Corynebacterium a été rapportée dans les demandes de brevet européen n° 0 064 680, 0 073 062, 0 073 063 et 0 073 064. Le polypeptide exprimé pourra être récupéré à partir du milieu de culture de ces Corynebacteriae, surtout de Corynebacteriae diphteriae, ou encore à partir des micro-organismes, dans les conditions décrites dans ces brevets.

Des ADN hybrides ou des vecteurs préférés de ce type sont encore ceux dont l'insérat contient en combinaison la séquence, basale ou mixte selon le cas, telle qu'elle a été définie plus haut et en phase avec la susdite séquence basale, une chaîne nucléotidique codant pour tout ou partie de la chaîne A ou de la chaîne B ou des deux à la fois, de la toxine diphtérique ou d'une anatoxine diphtérique. Un micro-organisme transformable par un tel vecteur est alors susceptible de synthétiser une protéine hybride, ayant une ou deux séquences peptidiques en commun avec la toxine diphtérique, d'une part, et une séquence peptidique correspondant à une partie au moins de la protéine déterminée dont la synthèse est recherchée. Il sera quelquefois fait référence dans ce qui suit à la protéine "tox-X", la partie "tox" correspondant à la toxine et "X" à la susdite protéine déterminée.

D'autres vecteurs préférés de l'invention sont ceux qui possèdent, soit immédiatement en amont de la susdite séquence basale ou mixte, soit en amont de la chaîne nucléotidique codant pour tout ou partie de la chaîne A ou de la chaîne B ou des deux à la fois, une séquence codant pour le site d'attachement du virus portant le gène "tox" et qui semble jouer un rôle essentiel au niveau de sa lysogénisation dans Corynebacterium diphteriae ou de l'insertion de l'ADN phagique dans le génome bactérien. En particulier des vecteurs préférés de l'invention contiennent la séquence :
GAAACCCCAGCTCATAGCATGTTTGAGCTGGGGTTTC.

De préférence aussi, et surtout lorsque la majeure partie des séquences nucléotidiques codant pour la chaîne A et/ou la chaîne B de la toxine diphtériques sont à déléter, il sera avantageux néanmoins de conserver la séquence codant pour la partie terminale hydrophile de la chaîne B, notamment la séquence en aval du site KpnI, à la position 1615 de la figure unique.

Ces vecteurs revêtent un intérêt particulier, lorsqu'ils sont aptes à transformer Corynebacterium et plus particulièrement un Corynebacterium diphteriae lysogénique pour le phage β, mais non lysogénisé par celui-ci. La transformation d'un tel micro-organisme par les vecteurs préférés de l'invention est alors susceptible de synthétiser et d'excréter dans leur milieu de culture la susdite protéine hybride, laquelle peut alors être isolée àpartir du milieu de culture. De telles protéines hybrides peuvent présenter une importance considérable, surtout lorsque cette protéine hybride est pratiquement la seule protéine excrétée dans le milieu de culture, à l'instar de la secrétion à titre principal de la toxine diphtérique dans des cultures de Corynebacterium diphteriae.Une telle protéine hybride peut alors présenter l'avantage essentiel qu'elle comporte à la fois une séquence polypeptidique contenant des déterminants ou sites antigéniques essentiels, caractéristiques de la protéine supplémentaire "X" dont la synthèse était recherchée, et une séquence de l'anatoxine diphtérique, d'une taille suffisante pour jouer le rôle de support ou "carrier" apte à conférer à ces protéines hybrides une immunogénicité susceptible de se manifester chez l'hôte auquel elle est administrée ou à la renforcer, pour une production d'anticorps actifs contre la susdite protéine supplémentaire "X".

Un domaine particulièrement préféré de l'invention est celui de la vaccination, notamment lorsque ladite protéine supplémentaire porte les sites ou déterminants antigéniques caractéristiques des protéines vaccinantes contre des micro-organismes, notamment des virus ou contre des toxines pathogènes déterminés.

De préférence, les séquences polypeptidiques que cette protéine possède en commun avec la toxine diphtérique correspondent à celles d'une toxine diphtérique spontanément non pathogène. Le cas échéant, la protéine hybride peut être détoxifiée par des méthodes d'inactivation classiques dans celui de la détoxification de la toxine diphtérique.

L'invention concerne naturellement plus particulièrement les micro-organismes transformés par les vecteurs ou ADN hybrides, tels qu'ils ont été définis ci-dessus, en particulier les E. coli ainsi transformés et les

10

Corynebacteriae ainsi transformés. De préférence, l'invention concerne des Corynebacterium diphteriae lysogéniques pour le phage β, mais non lysogénisés par le phage β naturel, ces Corynebacterium diphteriae étant cependant transformés par un ADN hybride ou vecteur du type susdit.

L'invention concerne encore le peptide S de formule :

MET SER ARG LYS LEU PHE ALA SER ILE LEU ILE GLY ALA LEU LEU GLY ILE

GLY ALA PRO PRO SER ALA HIS ALA

L'utilisation du système vecteur selon l'invention - Corynebacterium diphteriae pour produire des protéines ou polypeptides hybrides, tels qu'ils ont été définis plus haut, est particulièrement avantageuse.En particulier la séquence S présente dans ces vecteurs modifiés doit permettre le passage de ces molécules hybrides à travers la paroi bactérienne, d'où la possibilité d'obvier à d'éventuelles contaminations bactériennes lors de la purification de la protéine susceptible d'être isolée à partir du milieu de culture. En outre, ces micro-organismes transformés peuvent assurer une production en continu de l'hybride, sans lyse bactérienne due à une accumulation du produit dans le cytoplasme. L'utilisation de Corynebacterium diphteriae comme micro-organisme producteur doit être particulièrement avantageuse, notamment lors de la production de principes vaccinants, surtout si l'on tient compte de ce que la toxicité et les effets cytologiques de ce micro-organisme sont bien maîtrisés.

D'autres caractéristiques de l'invention apparaîtront encore au cours de la description qui suit des conditions dans lesquelles les parties caractéristiques de l'ADN selon l'invention ont été identifiées et des conditions dans lesquelles les séquences d'ADN des vecteurs de l'invention peuvent être mises en oeuvre pour la transformation de micro-organismes, en vue de la production de protéines hybrides. Cette description sera encore faite en rapport avec la figure unique, dans laquelle sont reproduites, d'une part, la séquence nucléotidique correspondant à une protoxine diphtérique associée aux éléments nécessaires à la régulation de la transcription de cette séquence et, d'autre part, la séquence polypeptidique correspondant à la protoxine diphtérique en question (c'est-à-dire à l'association "peptide S" - toxine).

1) Isolement d'un fragment du génome du phage $\beta_c 228$ codant pour la protoxine diptérique correspondante, c'est-à-dire un polypeptide comprenant le peptide signal S et les chaînes A et B de la toxine correspondante.

Le phage $\beta 228$ a été induit par traitement aux U.V. et à la NG d'une culture de <u>Corynebacterium diphteriae</u> $C_7 \beta 228$.

Le génome, préalablement isolé du phage $\beta_c 228$ apparu dans une plage claire est traité par l'enzyme de restriction <u>Bam</u>HI. Les fragments obtenus sont séparés par électrophorèse en gel d'agarose et transférés en partie sur un filtre de cellulose. Des essais d'hybridation <u>in situ</u> avec un ADN radioactif complémentaire de l'ARN messager et correspondant à la toxine diphtérique ont permis la détection d'un fragment <u>Bam</u>HI de 3,9 kb qui s'était hybridé avec la sonde. Celle-ci avait elle-même été préparée par la technique décrite par TAYLOR et Coll. (Biochem. Biophys. <u>Acta</u> 442, 324-330 (1976)).

Les fragments <u>Bam</u>HI correspondants encore contenus dans le gel sont alors extraits de celui-ci par électro-élution en sac à dialyse, concentrés et purifiés.

2) Clonage du fragment <u>Bam</u>HI de 3,9 kb et expression de celui-ci dans <u>E. coli</u>.

Le fragment <u>Bam</u>HI de 3,9 kb et le plasmide pBR322, préalablement linéarisé par l'enzyme de restriction <u>Bam</u>HI, ont été traités par l'ADN-ligase du phage T4. Le mélange des fragments ligaturés a été utilisé pour transformer des souches de <u>E. coli</u> transformables, telles que <u>E. coli</u> K12 MM294, au sein d'un milieu LB.

Les colonies de bactéries résistant à l'ampicilline et sensibles à la tétracycline sont alors sélectionnées. Des plasmides ainsi modifiés, désignés sous les expressions pTD44 et pTD76 contenant les fragments de 3,9 kb

13

insérés dans le site BamHI de pBR322 dans des sens respectivement opposés ont été isolés.

Des bactéries portant ces plasmides sont alors cultivées dans un milieu contenant de l'ampicilline, jusqu'à atteindre un niveau mesurable par le degré d'absorption d'une radiation de longueur d'onde de 600 nanomètres par lesdites cultures de 0,8 ($A_{600}$ = 0,8).

Les bactéries ont été séparées de leur milieu de culture, lavées et soumises à un traitement de rupture de leurs parois, après traitement au lysozyme. La présence dans les lysats de ces cellules d'une protéine correspondant à la protoxine diphtérique (CRM 228) a été détectée par un test d'immunoprécipitation à l'aide d'un antisérum de lapin antitoxine diphtérique par la méthode ELISA. La quantité de toxine synthétisée a été de l'ordre de 50 à 100 ng/ml de culture. Aucune immunoprécipitation n'a pu être observée dans des conditions semblables et mettant en jeu des lysats de bactéries non transformées.

Comme on l'a déjà observé, la production de la toxine a été constatée dans toutes les cultures transformées, indépendamment de l'orientation du fragment BamHI de 3,9 kb dans le plasmide récepteur.

Des résultats semblables ont été observés en mettant en oeuvre un plasmide pTD134 contenant à titre d'insérat un fragment BamHI semblable de 3,9 kb obtenu à partir d'un mutant spontané d'un phage $\beta_c$228 isolé d'une plage de lyse claire apparue spontanément dans une culture de $C_7\beta$228 non traitée aux U.V. et à la NG .

14

3) Analyse séquentielle des parties du fragment d'ADN selon l'invention.

L'analyse séquentielle de nucléotides a été réalisée sur le fragment BamHI de 3,9 kb réextrait de pTD134. L'analyse a porté sur plus de 95 % des deux brins de l'ADN par les techniques de MAXAM-GILBERT (Meth. Enzym. 65, 499-560 (1980)).L'analyse de séquence de la région contenant le promoteur et la séquence S ·a également été réalisée · par la méthode de F. SANGER et Coll. (J. Mol. Biol. 143, 161-178 (1980)), cela aussi pour le plasmide pTD134.

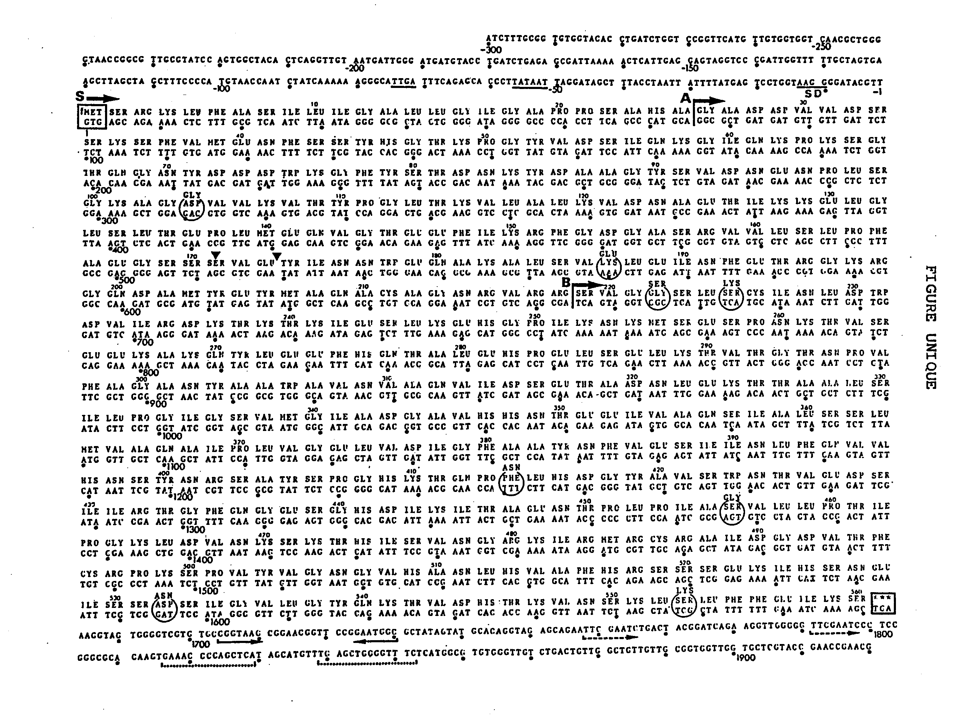

La structure séquentielle de l'ADN et, par voie de conséquence, des polypeptides pour lesquels code cet ADN sont représentés dans la figure 1. La numérotation des nucléotides débute avec le premier nucléotide de la séquence traduite. Chaque dixième nucléotide est marqué par des points dans les séquences aussi bien codantes que non codantes. La séquence d'aminoacides déduite de la phase de lecture ouverte est indiquée au-dessus de la séquence de nucléotides. Les aminoacides de cette séquence sont également numérotés de dix en dix. Les débuts respectifs du peptide signal S et des fragments A et B sont indiqués par des flèches. Les codons d'initiation et d'arrêt sont à l'intérieur de rectangles.

Ceux des aminoacides du polypeptide CRM228 qui se trouvent ainsi représentés, qui diffèrent des acides correspondants dans la toxine sauvage, dans la mesure où des structures peptidiques de parties de celle-ci étaient déjà connues, sont entourés d'un cercle. Les acides aminés correspondants de la toxine sauvage sont indiqués immédiatement au-dessus des acides aminés particuliers en question.

Les déterminations des acides aminés C-terminaux de la chaîne A et de la chaîne B, respectivement de la toxine, ont été déduites des comparaisons qui ont pu être faites entre les résultats de l'analyse de séquences du clone étudié et des séquences partiellement connues dont on disposait déjà antérieurement en ce qui concerne la constitution de la toxine diphtérique. La phase de lecture ouverte en amont de la séquence nucléotidique codant pour la chaîne A se termine au nucléotide -21 (fig. 1). L'interruption de ce cadre de lecture ouvert en l'absence d'un codon d'initiation ATG conduit à la conclusion plus que vraisemblable que la traduction est initiée au niveau du codon GTG qui se situe à l'extrémité amont de la phase de lecture. Il en résulte la constitution du peptide signal, dont la formule a déjà été indiquée plus haut. Il est à remarquer que ce peptide signal comporte un certain nombre d'éléments qui se retrouvent dans des peptides signaux déjà connus. En particulier, il comprend des résidus arginyle et lysyle au niveau des troisième et quatrième acides aminés du peptide S. Son extrémité C-terminale est formée de résidus histidyle et alanyle. Le site de clivage entre le peptide signal et le fragment A, notamment au niveau des acides aminés alanyle et glycyle, est également compatible avec des observations qui ont déjà été faites à propos d'autres séquences signal décrites dans la littérature.

La région codante pour le peptide S est précédée par des séquences similaires à celles trouvées en amont des gènes de E. coli. Ce sont :

(i) une séquence "Shine-Dalgarno" AAGG, située 9 paires de bases (pb) avant le premier codon traduit,

(ii) une séquence "TATAAT" tout à fait identique à la séquence consensus "-10" des promoteurs de E. coli et

(iii) une séquence "TTGATT" semblable à la séquence consensus "-35" (TTGACA) de ces mêmes promoteurs.

La région TATAAT ("Pribnow box") fait partie d'une séquence

16

répétée inversée de 9 pb qui joue peut-être un rôle dans la régulation de l'expression de la toxine, qui est sous le contrôle du fer dans Corynebacterium diphteria.

Dans la séquence nucléotidique située en aval du gène, un site de terminaison de la transcription semble être inclus dans une séquence répétée inversée de 7 nucléotides. Plus en aval, une autre structure secondaire est possible ; elle est composée d'une séquence répétée inversée de 14 pb de long, interrompue par 9 nucléotides. Cette structure est probablement le site d'attachement, att P, à l'aide duquel le phage β s'intègre lors de la lysogénèse de Corynebacterium diphteria. Ce site att P présente une structure similaire à celle démontrée pour le phage λ .

Partant de la connaissance ainsi acquise de la structure de la séquence nucléotidique codant pour la protoxine diphtérique, il a encore été possible de confirmer que l'expression de la séquence codante dans E. coli était bien sous le contrôle d'éléments de régulation et d'initiation de la transcription et de la traduction appartenant au fragment BamHI de 3,9 kb des insérats des plasmides selon l'invention.

La démonstration en a été faite en clonant le fragment HaeIII-HaeIII de 360 paires de bases contenant le promoteur et les 18 premiers codons de la séquence signal préalablement isolée à partir du gène tox cloné dans le plasmide pTD134, ce fragment ayant au préalable été placé, par recombinaison in vitro, devant le gène de la β-galactosidase dont avaient été délétés son propre promoteur et la séquence codant pour les huit premiers acides aminés de la β-galactosidase.

La construction a été faite de manière à respecter la phase de lecture au site de fusion entre la séquence signal et la β-galactosidase.

Le susdit fragment a été inséré dans le site SmaI, unique, situé au début du gène β -galactosidase du plasmide pSKS107 décrit par CASADABAN et Coll. (1980) et qui porte le gène codant pour la β-galactosidase à l'exception des parties délétées sus-indiquées. Le plasmide ainsi obtenu, nommé pTG113, a été sélectionné sur boite MacConkey indicatrice du lactose. Les clones produits après transformation de la souche E. coli MC1000, lac⁻, par le plasmide pTG113, sont rouges et démontrent la présence d'une activité β-galactosidase. Cette activité est portée par la protéine hybride "peptide - β-galactosidase". Elle a été dosée in vitro par réaction enzymatique sur l'O-nitrophényl-β-D galactoside. Les résultats obtenus montrent que le clone pTG113 possède une activité égale à environ 20-25 % de l'activité détectée dans des souches sauvages de E. coli.

Ces résultats démontrent que le promoteur de la toxine diphtérique cloné dans le plasmide pTD134 contrôle l'expression de ce gène dans E. coli. Ce même promoteur dans le plasmide pTG113 permet l'expression à un bon niveau d'une protéine hybride"peptide S-tox -β -galactosidase" dans E. coli.

On peut dans des conditions semblables produire une protéine hybride contenant un polypeptide, en commun avec une protéine déterminée, lorsque l'on remplace dans le système qui vient d'être décrit le gène codant pour la β -galactosidase par une séquence d'ADN codant pour ledit peptide. Le peptide hybride peut alors être récupéré à partir de E. coli, après rupture des parois de celui-ci.

Les vecteurs modifiés préférés de l'invention comportent, on l'a vu, de préférence tout ou partie des séquences codant pour les chaînes A et B de la toxine (ou anatoxine) diphtérique. Bien entendu, des parties importantes de ces séquences nucléotidiques peuvent être

18

délétées. D'une façon générale, il sera néanmoins préféré d'en conserver une partie importante, pour les raisons qui ont déjà été indiquées.

On décrit ci-après la préparation d'un gène recombinant codant pour une protéine hybride "tox-fragment de HBs".

Le site nucléotidique reconnu par l'enzyme de restriction KpnI (position 1615) a été choisi pour insérer dans le gène tox228 de pTD134 un fragment d'ADN codant pour une partie de l'antigène HBs, dont sont connues les propriétés immunogènes à l'égard du virus de l'hépatite virale B.

Ce fragment de 130 nucléotides de long (HBs2) provient de la digestion du gène S de HBV par les enzymes de restriction, BamHI et BstNI, aux positions nucléotidiques 488 et 618 vis-à-vis de la position 0 du site EcoRI de DNA-HBV.

Ce fragment a été traité par une enzyme exonucléolytique pour former des extrémités franches. Aux extrémités du fragment ainsi obtenu ont été accolés, en présence d'une enzyme ligase du phage T4, deux oligonucléotides obtenus par synthèse chimique, dont la séquence primaire avait été programmée pour être reconnue par KpnI. Le fragment ainsi modifié a été soumis à l'action de Kpn pour reformer des extrémités cohésives KpnI. Le fragment finalement obtenu et le plasmide pTD134, préalablement linéarisé par l'enzyme de restriction Kpn, ont été traités par une ADN-ligase du phage T4. Le plasmide formé (pTH1) contenant un gène fusionné tox228-HBs2 a été utilisé pour transformer E. coli. La protéine hybride formée, obtenue à partir du lysat de E. coli, est ensuite étudiée sur le plan de ses propriétés immunologiques, pour ses capacités à réagir notamment dans un test ELISA avec un sérum antidiphtérique et avec un sérum anti-HBs.

Dans ce qui précède, on a surtout envisagé les applications éventuelles de la protéine hybride formée dans le domaine de la vaccination. L'invention ne se limite évidemment pas à ce type d'application. En particulier, l'invention concerne églement l'application du vecteur modifié par un insérat contenant une séquence recombinante SA-Y, dans laquelle S et A auraient les significations mentionnées plus haut, et Y coderait pour un polypeptide spécifiquement reconnu par un récepteur membranaire spécifique, porté par des cellules tumorales. Par exemple, ce peptide correspondrait à l'un de ceux décrits par I.S. TROWBRIDGE et Coll.(Nature, 294, 171-173 (1981)). Plus généralement, Y peut être constitué par tout autre polypeptide susceptible d'être mis en jeu dans l'étude du comportement de la chaîne A de la toxine diphtérique vis-à-vis de différents types de cellules, par exemple dans les conditions décrites par D.L. SIMPSON et Coll. (Cell. 29, 469-473 (1982)).

Comme il va de soi et comme il résulte d'ailleurs déjà de ce qui précède, l'invention ne se limite nullement à ceux de ses modes d'application et de réalisation qui ont été plus spécialement envisagés ; elle en embrasse au contraire toutes les variantes ; en particulier, l'invention concerne toute séquence nucléotidique ou tout ADN hybride se distinguant de ceux qui ont été plus particulièrement décrits, soit par des délétions ou additions de fragments d'ADN supplémentaires, soit par des substitutions de codons par d'autres, dans la mesure où les polypeptides résultant de ces délétions, additions ou modifications conservent sensiblement

20

les mêmes propriétés. Les ADN qui en résultent doivent naturellement être considérés comme des équivalents entrant dans le champ de protection conféré à l'invention par les revendications qui suivent.

Il en sera encore ainsi plus particulièrement du peptide signal, lequel peut éventuellement être modifié par séparation de certains de ses aminoacides, ou remplacement éventuel de certains de ses aminoacides par d'autres aminoacides ne modifiant pas sensiblement les propriétés du peptide S, notamment en ce qui concerne sa capacité à promouvoir l'excrétion de la protéine hybride ou non, codée par la séquence d'ADN nucléotidiques associée à celle auquel correspond ce peptide S modifié.

Entrent également dans le champ de l'invention revendiquée les séquences d'ADN dans lesquelles la séquence S serait associée à d'autres promoteurs susceptibles d'être reconnus par Corynebacterium diphteriae.

En outre, dans ce qui précède, il a surtout été fait état du gène de la toxine diphtérique ou de fragments de celui-ci. Il va de soi que doivent être considérées comme des équivalents entrant également dans le champ de protection des revendications toutes autres formes d'ADN ou de fragments d'ADN capables de coder pour des polypeptides identiques ou ayant des propriétés semblables. On mentionnera à titre d'exemple les séquences de cADN susceptibles d'être obtenues par recopie enzymatique de l'ARN messager correspondant à une protoxine diphtérique. Ce cADN peut alors être mis sous le contrôle d'un promoteur contenu dans une séquence d'ADN distinct par des méthodes de recombinaison génétique, bien connues des spécialistes.

La souche Corynebacterium diphteriae $C_7\beta228$ a été déposée à la C.N.C.M. sous le n° I-236 le 28 juillet 1983.

21
REVENDICATIONS

1 - Fragment d'ADN contenant au moins 75 paires de nucléotides et en contenant au plus 1000, notamment au plus 375, caractérisé en ce qu'il contient une séquence basale de nucléotides codant pour la séquence peptidique suivante :

MET SER ARG LYS LEU PHE ALA SER ILE LEU ILE GLY ALA LEU LEU GLY ILE

GLY ALA PRO PRO SER ALA HIS ALA

2 - Fragment d'ADN selon la revendicatiop 1, caractérisé en ce qu'il contient la séquence basale de nucléotides :

GTG AGC AGA AAA CTG TTT GCG TCA ATC TTA ATA GGG GCG CTA CTG GGG ATA

GGG GCC CCA CCT TCA GCC CAT GCA|

3 - Fragment d'ADN selon la revendication 2, caractérisé en ce qu'il contient une séquence mixte comportant la susdite séquence basale et une seconde séquence, en amont de la précédente, vis-à-vis de la direction de lecture, cette seconde séquence contenant un promoteur, en sorte que la première soit placée sous le contrôle de ce promoteur.

4 - Fragment d'ADN selon la revendication 3, caractérisé en ce que la susdite seconde séquence contient la structure nucléotidique suivante :

TTGA TTTCAGAGCA

CCCTTATAAT TAGGATAGCT TTACCTAATT ATTTTATGAG TCCTGGTAAG GGGATACGTT
-50                                                              -1

5 - Fragment d'ADN selon la revendication 4, caractérisé par la séquence :

```
ATCTTTGCGG TGTGGTACAC CTGATCTGGT CCGGTTCATG TTGTGGTGGT CAACGCTGGG
-300                                                   -250

GTAACCGGCG TTGCGTATCC AGTGGCTACA CTCAGGTTGT AATGATTGGG ATGATGTACC
                                             -200

TGATCTGAGA CCGATTAAAA ACTCATTGAG CAGTAGGTCC CGATTGGTTT TTGCTAGTGA
                             -150

AGCTTAGCTA GCTTTCCCCA TGTAACCAAT CTATCAAAAA AGGGCATTGA TTTCAGAGCA
                   -100

CCCTTATAAT TAGGATAGCT TTACCTAATT ATTTTATGAG TCCTGGTAAG GGGATACGTT
           -50                                                  -1

GTG AGC AGA AAA CTG TTT GCG TCA ATC TTA ATA GGG GCG CTA CTG GGG ATA

GGG GCC CCA CCT TCA GCC CAT GCA
```

6 - ADN hybride contenant le fragment selon l'une
quelconque des revendications 1 à 5, associé à une séquence d'ADN hétérologue vis-à-vis de l'ADN du phage β .

7 - ADN hybride selon la revendication 6, caractérisé en ce qu'il consiste en un vecteur apte à transformer un micro-organisme déterminé et comportant un promoteur susceptible d'être reconnu par ledit micro-organisme
déterminé, ce vecteur étant modifié par l'insertion dans
un site en amont de ce promoteur de la séquence basale définie dans la revendication 1 ou la revendication 2, celle-
ci étant alors placée sous le contrôle de ce promoteur.

8 - ADN hybride selon la revendication 6, caractérisé en ce qu'il consiste en un vecteur apte à transformer
un E.coli ou un Corynebacterium   ce vecteur contenant
la séquence mixte de nucléotides définie dans le revendication 4 ou la revendication 5.

9 - ADN hybride selon l'une quelconque des revendications 6 à 8, caractérisé en ce qu'il contient également, en aval de la susdite séquence, selon le cas basale ou mixte, et en phase avec celle-ci, un insérat hétérologue vis-à-vis de l'ADN du phage β.

10 - ADN hybride selon l'une quelconque des revendications 6 à 8, contenant en combinaison la séquence, selon le cas basale ou mixte, et, en phase avec ladite séquence basale, une chaîne nucléotidique codant pour tout ou partie de la chaîne A ou de la chaîne B, ou des deux à la fois, de la toxine ou d'une anatoxine diphtérique.

11 - ADN hybride selon la revendication 10, caractérisé en ce qu'il contient, inséré dans la susdite chaîne nucléotidique et en phase avec la susdite séquence de base, un insérat codant pour une séquence protéique hétérologue vis-à-vis de l'ADN du phage β.

12 - Micro-organisme transformé par un fragment d'ADN ou par l'ADN hybride selon l'une quelconque des revendications 1 à 11.

13 - Micro-organisme selon la revendication 12, caractérisé en ce qu'il est dérivé de E. coli.

14 - Micro-organisme selon la revendication 12, caractérisé en ce qu'il est dérivé d'un Corynebacterium lysogénique pour le phage β, mais non lysogénisé par ce dernier.

15 - Procédé d'obtention d'une protéine hybride comprenant une séquence peptidique déterminée, caractérisé par la transformation par un ADN hybride selon l'une quelconque des revendications 9 à 11 d'un micro-organisme transformable par ce fragment d'ADN ou cet ADN hybride, celui-ci contenant également un insérat codant pour ladite séquence peptidique déterminée.

16 - Procédé selon la revendication 15, caractérisé en ce que le micro-organisme transformable est constitué par un Corynebacterium diphteriae lysogénique pour le phage β mais non lysogénisé par celui-ci, et en ce que la

24

séquence polypeptidique déterminée susdite est récupérée dans le milieu de culture du micro-organisme.

17 - Peptide de formule :

MET SER ARG LYS LEU PHE ALA SER ILE LEU ILE GLY ALA LEU LEU GLY ILE

GLY ALA PRO PRO SER ALA HIS ALA

0133403

FIGURE UNIQUE